# EUROPEAN PATENT APPLICATION

(11) **EP 4 190 379 A1**
(43) Date of publication of application: **07.06.2023**
(21) Application number: 21212447.3
(22) Date of filing: 06.12.2021
(51) Int. Cl.: A61M 5/315, A61J 1/00

(54) **STOPPER FOR A SYRINGE HAVING ANTI-STICKING FEATURES**

(71) Applicant: Becton, Dickinson and Company, Franklin Lakes, NJ 07417-1880 (US)
(72) Inventor: FREMON, Benoit, 38410 Saint Martin d'Uriage (FR); VAXELAIRE, Jérémie, 38000 Grenoble (FR); POUGET, Gaëlle, 38100 GRENOBLE (FR); RODRIQUEZ SAN JUAN, Nestor, Hamburg, NJ 07419 (US); PRAIS, Alfred, W., New Jersey 07421 (US)
(74) Representative: Germain Maureau

(57) **Abstract**

A stopper for a syringe including a body having a tail adapted for attachment to a plunger of the syringe, and a head, the head having a outer surface that is convex, such that the outer surface is curved with an apex of the curvature extending away from a plane orthogonal to the longitudinal axis of the stopper and comprising an outer perimeter of the outer surface and the apex of the outer surface of the head is higher than the outer perimeter of the outer surface when the stopper is viewed from a side in a direction perpendicular to the longitudinal axis of the stopper. The stopper may optionally include a plurality of protrusions extending from an outer surface of the head. Also, a syringe including such a stopper.

## Description

### BACKGROUND OF THE INVENTION

### Field of the Invention

The present invention is directed to a stopper for a syringe, the stopper having a head with a convex outer surface and optionally anti-sticking features extending from the convex outer surface of the head.

### Description of Related Art

With prior art stoppers for syringes, during storage or assembly, the outer surface of the head of one stopper can become stuck to the outer surface of the head of another stopper. Sticking can occur either during storage or shipping, when the stoppers are packaged in plastic bags that may be evacuated of air and stacked or when the stoppers are conveyed in a vibrating bowl prior to being assembled into the syringe. Those issues can prevent processing of the stoppers. Thus, stopper to stopper stickiness occurrence and adhesion strength must be minimized to allow processability and reduce scrap.

Close contact, which will be referred to herein as the apparent contact area, between two surfaces of the stoppers can occur when an external force is applied to the surfaces of the stoppers that are in contact with one another. This force can be applied to packaged stoppers, when air is evacuated from the bag containing the stoppers to increase packaging density, and when bags of stoppers are packaged in cardboard boxes that are stacked together on a pallet for shipping, the external force can be magnified.

Stoppers are typically made from thermoplastic material or rubber. For non-coated stoppers, the soft stopper material having a smooth surface without an outer silicone layer or with an inadequate amount of silicone can be prone to rubber-to-rubber sticking when the stoppers are in close contact.

While the provision of a silicone layer on the stopper can reduce rubber-to-rubber sticking, the presence of the silicone on the head of the stoppers can also lead to sticking of the heads of the stoppers to one another. For coated stoppers, where a silicone layer and/or a film such as for example polytetrafluoroethylene (PTFE) or ethylene tetrafluoroethylene (ETFE) are applied on the head of the stopper, it is postulated that sticking of stoppers to one another occurs by capillary bridges. The higher the amount of silicone, the more likely the outer surfaces of the heads of the stoppers are to stick to one another.

Vent tube assembly processing when the stoppers are assembled into the syringes requires silicone on the stopper's surface to avoid stopper degradation. Therefore, when the stoppers are assembled into a syringe using vent tube assembly processing, lowering the silicone quantity is not the optimized option to decrease the sticking of the heads of the stoppers to one another.

Further, when the outer surfaces of the heads of the stoppers are flat, the large contact area facilitates the sticking between the stoppers.

With these factors in mind, inventive stoppers having a convex head and optional anti-sticking features were developed in order to reduce the sticking of stoppers to one another during assembly and storage.

### SUMMARY OF THE INVENTION

The present invention is directed to a stopper for a syringe comprising a body having a tail adapted for attachment to a plunger rod and a head. The outer surface of the head of the stopper is convex. As used herein, a convex outer surface of the head of the stopper is one in which the outer surface of the head of the stopper is curved, such that the apex of the curvature extends away from a plane which is orthogonal to the longitudinal axis of the stopper and comprising the outer perimeter of the outer surface, i.e., a distance between the apex of the outer surface of the head and the plane is greater than the distance between the portion of the outer surface of the head adjacent the outer perimeter of the outer surface of the head and the plane when the stopper is viewed from the side in a direction perpendicular to the longitudinal axis of the stopper.

A radius of curvature (r) of the outer surface of the head of the stopper may be greater than or equal to a maximum diameter (D) of the head of the stopper and less than or equal to 20 times the maximum diameter (D) of the head of the stopper. A ratio of the radius of curvature (r) of the outer surface of the head of the stopper to the maximum diameter (D) of the head of the stopper (r/D) may be 1-20, preferably 1-4, for example, 1.05-2.20.

The stopper may further comprise a coating applied to at least a portion of the stopper that will contact drug contained within the syringe.

The stopper may optionally include anti-sticking features extending from the outer surface of the head of the stopper.

The anti-sticking features may comprise a plurality of protrusions extending outwardly from the outer surface of the head of the stopper, wherein the protrusions have preferably a substantially triangular shape, and more preferably, a shape that is substantially an isosceles triangle, with a vertex corresponding to the vertex angle of the triangular shape of the protrusion, a base corresponding to the base of the triangular shape of the protrusion, and two sides extending between the vertex and the base, wherein the vertex of each protrusion is adjacent the center of the outer surface of the head of the stopper and the base of each protrusion is adjacent the outer perimeter of the outer surface of the head of the stopper. A space may be provided between the bases of the protrusions and the outer perimeter of the outer surface of the head of the stopper and/or a space defined by the vertex of the protrusions may be provided at a center of the outer surface of the head of the stopper. The protrusions may be evenly distributed around a circumference of the outer surface of the head of the stopper such that corresponding substantially triangular spaces are provided between adjacent protrusions, and an angular distance θ_{S} occupied by the spaces may be less than the angular distance θ_{P} occupied by one of the protrusions. The sum of the angular distances θ_{S} occupied by the spaces may be less than the sum of the total angular distances θ_{P} occupied by the protrusions. Preferably, the angular distance θ_{P} occupied by one of the protrusions may be 40-80° and/or the angular distance occupied by one protrusion and one space (θ_{P}+ θ_{S}) may be 60-130°. A radial length of each protrusion may be 70-90% of an outer diameter (d) of the outer surface of the head of the stopper.

An outer surface of the protrusions may be flat or concave or convex with a curvature extending from the vertex to the base of the protrusion, and/or the outer surface of the protrusions may be flat or concave or convex with a curvature extending from one side of the protrusion to the other side of the protrusion. The curvature extending from the vertex to the base of the protrusion may be defined by a fillet portion at the vertex of the protrusion having a first radius of curvature, a fillet portion at the base of the protrusion having a second radius of curvature, and an upper surface extending between the fillet portion at the vertex and the fillet portion at the base that is flat or has a third radius of curvature, wherein the first radius of curvature and the second radius of curvature may be equal and/or the third radius of curvature may be greater than the first radius of curvature and the second radius of curvature. The curvature of the fillet portion of the vertex and/or the curvature of the fillet portion of the base may be concave, i.e., rounded inwardly, or convex, i.e. rounded outwardly. Preferably, the curvature of the fillet portion of the vertex and the fillet portion of the base have a convex curvature where the fillets abut the upper surface that changes to a concave curvature as the fillet approaches the outer surface of the head of the stopper.

The curvature extending from one side of the protrusion to the other side of the protrusion may be defined by a fillet portion at one side of the protrusion having a first radius of curvature, a fillet portion at the other side of the protrusion having a second radius of curvature, and the upper surface extending between the fillet portions at the sides of the protrusion being flat or having a third radius of curvature, wherein the first radius of curvature and the second radius of curvature may be equal and/or the third radius of curvature may be greater than the first radius of curvature and the second radius of curvature. The curvature of the fillet portions of the sides of the protrusion may be concave, i.e., rounded inwardly, and/or convex, i.e. rounded outwardly. Preferably, the fillet portions will have a convex curvature where the fillets abut the upper surface that changes to a concave curvature as the fillet approaches the outer surface of the head of the stopper.

Alternatively, the optional anti-sticking features may be a plurality of concentric annular protrusions extending outwardly from the outer surface of the head of the stopper and separated by interspaces. An outer surface of the annular protrusions has a convex curvature in a radial direction while the interspaces between adjacent annular protrusions have a concave curvature in a radial direction. A radius of curvature of the interspaces may be greater than or equal to the radius of curvature of the outer surface of the annular protrusions, and is preferably greater than the radius of curvature of the outer surface of the annular protrusions. A space provided between the outermost annular protrusion and the outer perimeter of the outer surface of the head of the stopper may be provided. The annular protrusions may be discontinuous and include at least one gap such that the annular protrusion comprises a plurality of sections.

The features of at least the head of the stopper and transitions between the features of at least the head of the stopper may be smoothly rounded.

The present invention is also directed to a syringe comprising a syringe body defining a chamber, a plunger rod at least partially received within the chamber, and a stopper as described above.

### BRIEF DESCRIPTION OF THE DRAWINGS

FIG. 1 is cross-sectional view of the inventive stopper within a syringe;
FIG. 2 is a perspective view of an inventive stopper according to the invention;
FIG. 3 is a top view of the inventive stopper of FIG. 2;
FIG. 4 is a cross section of the inventive stopper along line A-A of FIG. 3;
FIG. 5 is a perspective view of the inventive stopper of FIG. 1 with the addition of anti-sticking protrusions added to the outer surface of the head of the inventive stopper;
FIG. 5A is a cross-sectional view along line 5A-5A in FIG. 5;
FIG. 6 is a top view of the inventive stopper of FIG. 5;
FIG. 7 is a cross section of the inventive stopper along line B-B of FIG. 6;
FIG. 8 is a perspective view of the inventive stopper of FIG. 1 with the addition of anti-sticking annular protrusions added to the outer surface of the head of the inventive stopper;
FIG. 9 is a top view of the inventive stopper of FIG. 8;
FIG. 10 is a cross section of the inventive stopper along line C-C of FIG. 9.
FIG. 11 is a perspective view of the inventive stopper of FIG. 1 with the addition of discontinuous anti-sticking annular protrusions added to the outer surface of the head of the inventive stopper;
FIG. 12 is a top view of the inventive stopper of FIG. 11;
FIG. 13 is a cross section of the inventive stopper along line D-D of FIG. 12;
FIG. 14A is a cross-sectional view of a stopper having a flat head at the distal end of a syringe body;
FIG. 14B is a cross-sectional view of a stopper having a convex head at the distal end of a syringe body; and
FIG. 14C is a cross-sectional view of a stopper having a convex head with annular protrusions at the distal end of a syringe body.

### DESCRIPTION OF THE INVENTION

As used herein, unless otherwise expressly specified, all numbers such as those expressing values, ranges, amounts or percentages may be read as if prefaced by the word "about", even if the term does not expressly appear. Any numerical range recited herein is intended to include all sub-ranges subsumed therein. For example, a range of "1 to 10" is intended to include any and all sub-ranges between and including the recited minimum value of 1 and the recited maximum value of 10, that is, all subranges beginning with a minimum value equal to or greater than 1 and ending with a maximum value equal to or less than 10, and all subranges in between, e.g., 1 to 6.3, or 5.5 to 10, or 2.7 to 6.1. Plural encompasses singular and vice versa. When ranges are given, any endpoints of those ranges and/or numbers within those ranges can be combined with the scope of the present invention. "Including", "such as", "for example" and like terms means "including/such as/for example but not limited to".

For purposes of the description hereinafter, spatial orientation terms, as used, shall relate to the referenced embodiment as it is oriented in the accompanying drawings, figures, or otherwise described in the following detailed description. As used herein, "distal" refers the end of the stopper comprising the head or an injection end of the syringe. In the cross-sectional drawings of the stopper, "distal" refers to the top end of the stopper. Similarly, a "distal direction" is a direction toward the head of the stopper or toward the injection end of the syringe. As used herein, "proximal" refers the end of the stopper comprising the tail or a plunger end of the syringe. In the cross-sectional drawings, "proximal" refers to the bottom end of the stopper. Similarly, a "proximal direction" is a direction toward the tail of the stopper or toward the plunger end of the syringe.

The present invention is directed to a stopper 10, 100, 200, 300 to be used in a syringe 12. The syringe 12 comprises a syringe body 14 and a plunger 16 to which the stopper 10, 100, 200, 300 is attached.

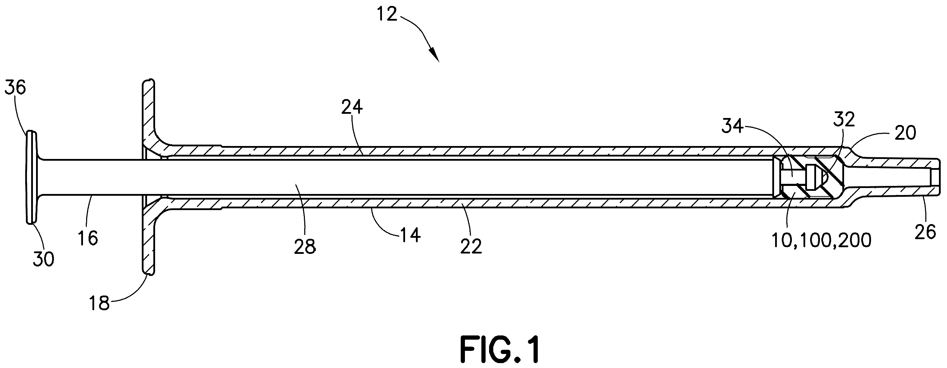

As shown in FIG. 1, the syringe body 14 comprises a proximal end 18, a distal end 20, and a sidewall 22 extending between the proximal end 18 and distal end 20. The sidewall 22 defines a chamber 24 adapted to receive a pharmaceutical composition. The chamber 24 is substantially cylindrical. A fitting 26 for attaching a cannula assembly to the syringe 12 extends in the longitudinal direction from the distal end 20 of the syringe body 14. The fitting 26 may be a luer lock or a luer slip. The cannula assembly may be permanently attached to the syringe 12, or alternatively, may be attached to the syringe 12 just before usage.

The syringe body 14 may be made from plastic or glass.

The plunger 16 comprises a plunger rod 28 having a proximal end 30 and a distal end 32, an engagement portion 34 extending from the distal end 32 of the plunger rod 28, and may include a thumb pad 36 extending radially outward from the proximal end 30 of the plunger rod 28 in a direction substantially perpendicular to the longitudinal axis of the plunger rod 28. At least a portion of the plunger rod 28 and the engagement portion 34 of the plunger rod 28 are contained within the chamber 24 of the syringe body 14. The engagement portion 34 of the plunger rod 28 is adapted for attachment to the stopper 10, 100, 200, 300. The plunger 16 is movable with respect to the syringe body 14 such that a distal force applied to the plunger 16 while holding the syringe body 14 stationary causes the engagement portion 34 of the plunger rod 28 and the stopper 10, 100, 200, 300 attached thereto to be displaced in a distal direction within the chamber 24 of the syringe body 14, and a proximal force applied to the plunger 16 while holding the syringe body 14 stationary causes the engagement portion 34 of the plunger rod 28 and the stopper 10, 100, 200, 300 attached thereto to be displaced in a proximal direction within the chamber 24 of the syringe body (14).

At least a portion of the stopper 10, 100, 200, 300 has a diameter that is equal to or larger than an inner diameter of the chamber 24 of the syringe body 14 thereby creating a sealing engagement between the portion of the stopper 10, 100, 200, 300 and the inner surface of the sidewall 22 of the syringe body 14.

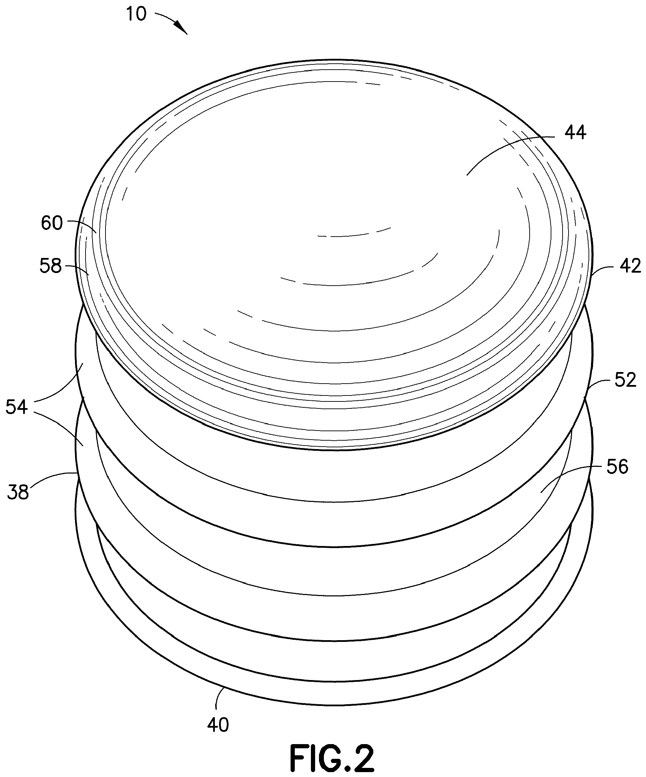

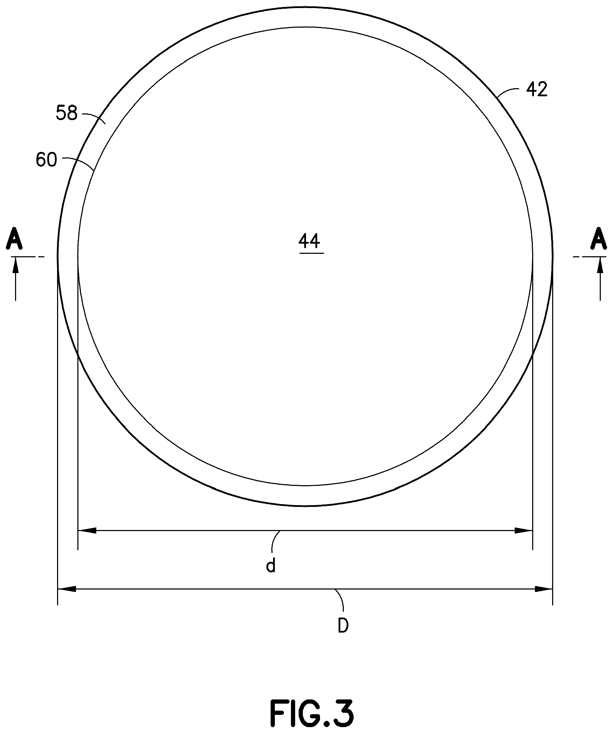

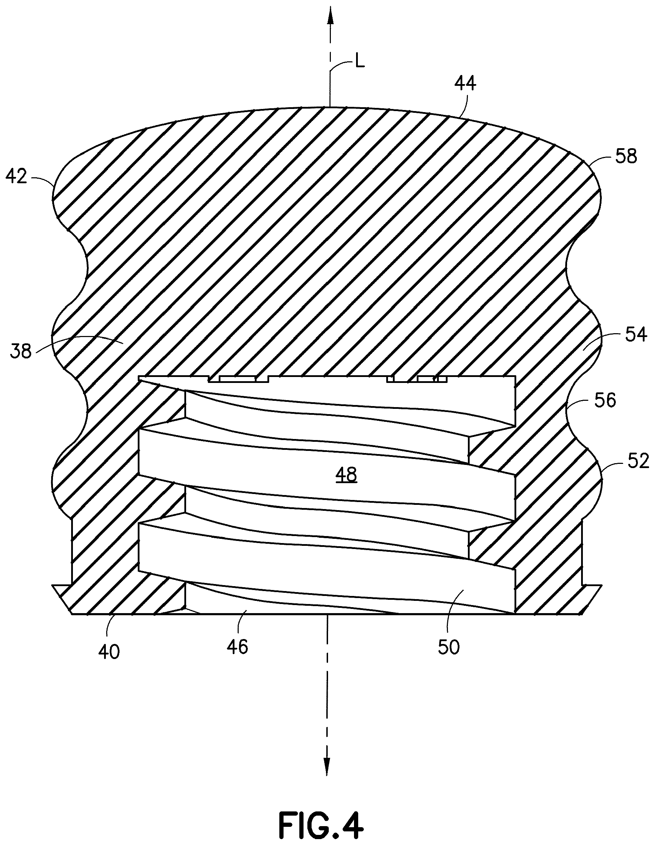

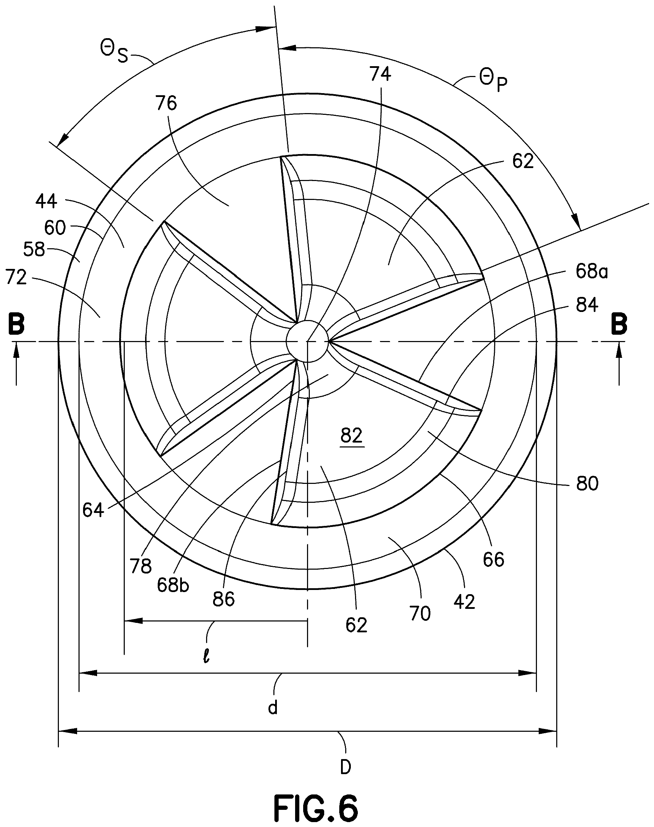

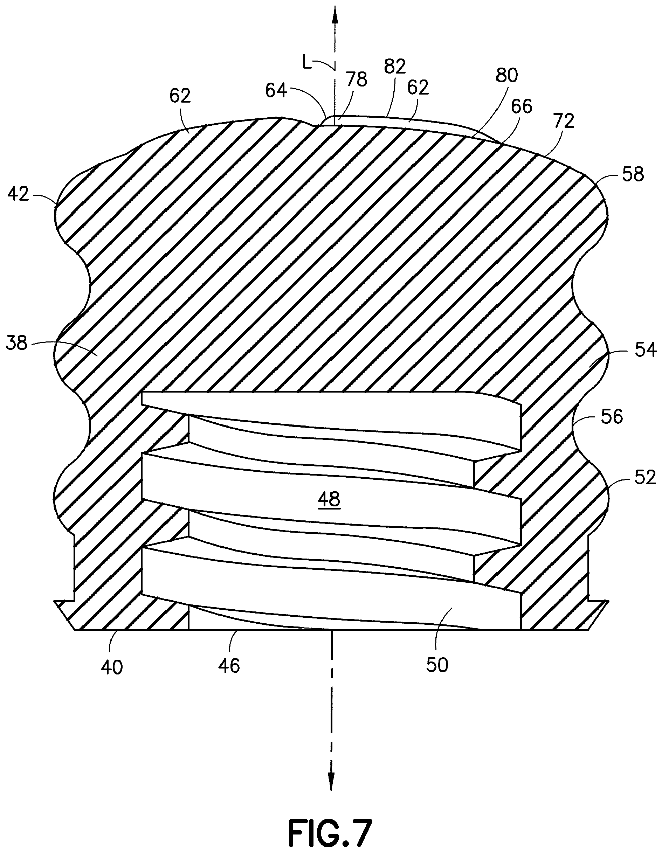

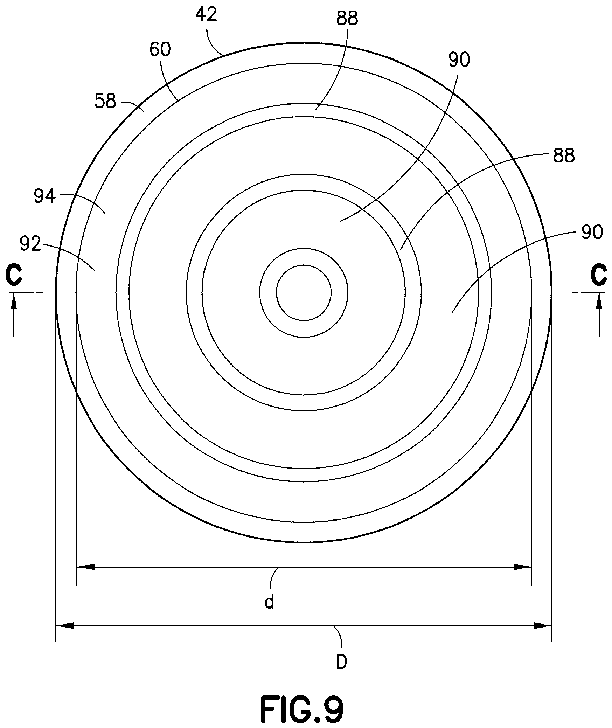

As shown in FIGS. 2-9, the stopper 10, 100, 200, 300 comprises a body 38 having a proximal tail or proximal tail end 40 adapted for attachment to the plunger rod 28 of the syringe 12 and a distal head or distal head end 42. The tail 40 includes an opening 46 directed towards a cavity 48 defined within the body 38. The opening 46 and the cavity 48 are adapted to receive and engage the engagement portion 34 of the plunger 16 thereby attaching the stopper 10, 100, 200, 300 to the plunger 16. The opening 46 of the cavity 48 has a diameter that is smaller than the outer diameter of the tail 40 of the stopper 10, 100, 200, 300. While the stoppers 10, 100, 200, 300 shown in the figures include the cavity 48, it is to be understood that the cavity 48 is optional and the plunger 16 may be attached to the stopper 10, 100, 200, 300 via other means, such that no cavity 48 is required. The inner sidewall of the cavity 48 may include threads 50 for attaching the stopper 10, 100, 200, 300 to the plunger rod 28.

The substantially cylindrical stopper body 38 optionally includes a plurality of annular ribs 54 around the circumference of the outer sidewall 52 of the stopper body 38. The ribs 54 may be evenly spaced from one another by grooves 56. The ribs 54 have a diameter that is greater than the diameter of the grooves 56.

The stopper 10, 100, 200, 300 may be made from any suitable compressible material including, but not limited to, natural rubber, synthetic rubber, and more particularly rubbers made from butyl, bromobutyl, chlorobutyl, silicone, nitrile, styrene butadiene, polychlororprene, ethylene propylene diene, fluoroelastomers, thermoplastic elastomers, and combinations and blends thereof, and is preferably made from butyl rubber, such as bromobutyl.

Optionally, the portion of the head 42 of the stopper 10, 100, 200, 300 and at least the outer surface 44 of the head 42 of the stopper 10, 100, 200, 300 that can potentially contact the pharmaceutical composition in the chamber 24 of the syringe 12 is coated with a coating that acts as a barrier between the stopper material and the pharmaceutical composition that occupies the chamber 24 of the syringe 12 and enables reduction of extraction and/or leaching of substances from the stopper material and infiltration of the pharmaceutical composition into the stopper material. The coating may be a fluoropolymer such as polytetrafluoroethylene (PTFE), ethylene tetrafluoroethylene (ETFE), expanded polytetrafluoroethylene (ePTFE), flurorinated ethylene propylene (FEP), polyvinylidene fluoride, polyvinylfluoride, perfluoropropylvinylether, perfluoroalkoxy polymers, tetrafluoroethylene (TFE), parylene, or non-fluoropolymers such as polyethylene, polypropylene, Parylene C, and Parylene F. Preferably the coating is ETFE. The coating may optionally extend around the outer edge of the outer surface 44 of the head 42 and along at least a portion of the outer sidewall 52 of the stopper 10, 100, 200, 300. The coating may also cover at least one lateral rib 54, preferably at least two lateral ribs 54 of the stopper 10, 100, 200, 300. The coating may be applied to the stopper material by the second molding step of a two-step molding process, calendaring, or spraying an emulsion layer containing a coating precursor with a reticulation step onto the stopper material that is later bonded with the stopper material. Reticulation may occur via thermal treatment and/or UV treatment.

A silicone layer for lubricity, decreased stickiness, and/or protection of the stopper 10, 100, 200, 300 during vent tube assembly processing may optionally be provided on at least a portion of the outer surface of the stopper 10, 100, 200, 300. Such silicone layers include, but are not limited to polydimethylsiloxane (PDMS) and 1000cP silicone, for example Dupont^{™} Liveo^{™} 360 medical fluid, GelestDMS-T23, or NuSil MED-361. The silicone layer may have an area density of 60 µg.cm⁻² or less, and may be 10-110 µg.cm⁻² when the stopper 10, 100, 200, 300 will be subjected to vent tube assembly processing during assembly of the stopper 10, 100, 200, 300 into the syringe 12 or may be less than 10 µg.cm⁻² when the stopper 10, 100, 200, 300 will be subjected to vacuum assembly processing during assembly of the stopper 10, 100, 200, 300 into the syringe 12.

In order to avoid tears in the coating and/or non-adhesion of the coating to the stopper material, sharp angles in the outer contour of the stopper body 38 are avoided, and transitions between features of the stopper 10, 100, 200, 300 are smoothly rounded. The transition of the outer perimeter 60 of the outer surface 44 of the head 42 of the stopper 10, 100, 200, 300 to the outer sidewall 52 of the stopper 10, 100, 200, 300, the ribs 54, and the grooves 56 may be rounded. Typically, the radius of curvature of the outer contour of the stopper body 38 may be between 2.0 and 27.2% of the maximal diameter of the head of the stopper; preferably between 2.5 and 18.5%, or even more preferably between 5.5 and 13.0%.

For example, for a stopper 10, 100, 200, 300 having a head 42 having a maximum diameter of 9.2 mm, the transition of the outer perimeter 60 of the outer surface 44 of the head 42 of the stopper 10, 100, 200, 300 to the outer sidewall 52 of the stopper 10, 100, 200, 300 may have a radius of curvature of 0.75 mm, the ribs 54 may have a convex radius of curvature of 0.6-0.9 mm, preferably 0.75 mm, and the grooves 56 may have a concave radius of curvature of 0.6-0.9 mm, preferably 0.75 mm. The radius of curvature of these features may be adjusted proportionately based on the maximum diameter of the head 42 of the stopper 10, 100, 200, 300.

With prior art stoppers where the outer surface of the head is flat, during storage and/or assembly, the outer surface of the head of one stopper can become stuck to the outer surface of the head of another stopper.

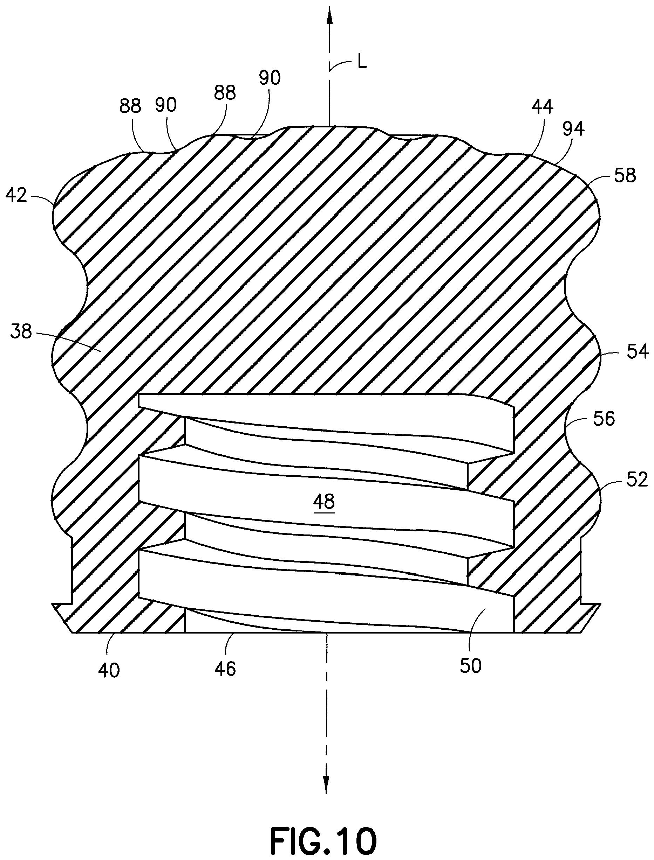

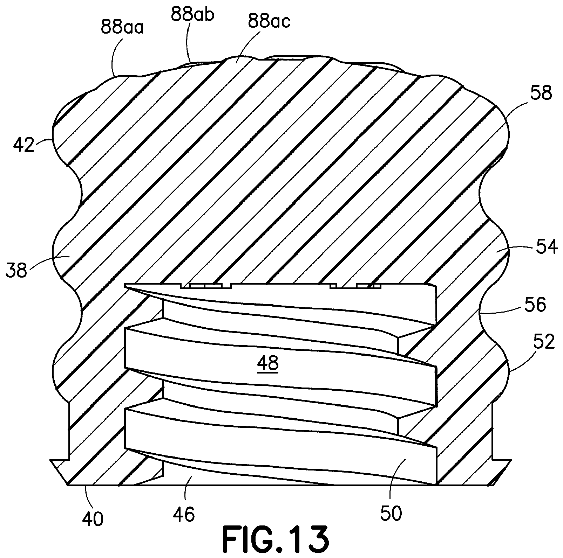

The outer surface 44 of the head 42 of the inventive stopper 10, 100, 200, 300 is convex with or without anti-sticking features extending from the outer surface 44 of the head 42. A convex outer surface 44 of the head 42 of the stopper 10, 100, 200, 300, as shown in the FIGS. 1-13, is one in which the outer surface 44 of the head 42 of the stopper 10, 100, 200, 300 is curved, such that the apex of the curvature extends away from a plane orthogonal to the longitudinal axis L of the stopper and comprising the outer perimeter 60 of the outer surface 44 of the head 42 of the stopper 10, 100, 200, 300, i.e., the distance between the apex of the outer surface 44 of the head 42 and the plane is greater than the distance between the portion of the outer surface 44 of the head 42 adjacent the outer perimeter 44 of the outer surface 44 of the head 42 and the plane when the stopper 10, 100, 200, 300 is viewed from the side in a direction perpendicular to the longitudinal axis L of the stopper 10, 100, 200, 300. The convex outer surface 44 of the head 42 of the stopper 10, 100, 200, 300 reduces the contact area between the outer surfaces 44 of the heads 42 of two stoppers 10, 100, 200 when the outer surfaces 44 of the heads 42 of the two stoppers 10, 100, 200, 300 come in contact with one another.

A radius of curvature r of the outer surface 44 of the head 42 of the stopper 10, 100, 200, 300 may be greater than or equal to a maximum diameter D of the head 42 of the stopper 10, 100, 200, 300 and less than or equal to 20 times the maximum diameter D of the head 42 of the stopper 10, 100, 200, 300. The ratio of the radius r of curvature of the outer surface 44 of the head 42 of the stopper 10, 100, 200, 300 to the maximum diameter D of the head 42 of the stopper 10, 100, 200, 300 (r/D) may be 1-20, and preferably 1-4, for example, 1.05-2.2. For example, the stopper 10, 100, 200, 300 may have a head 42 with a maximum diameter D of 9.2 mm and the outer surface 44 of the head 42 of the stopper 10, 100, 200, 300 may have a radius r of curvature of 12 mm, such that the ratio of the radius r of curvature of the outer surface 44 of the head 42 of the stopper 10, 100, 200, 300 to the maximum diameter D of the head 42 of the stopper 10, 100, 200, 300 (r/d) is 1.3.

The head 42 of the stopper 10, 100, 200, 300 may optionally include an annular fillet portion 58 surrounding the outer perimeter 60 of the convex outer surface 44 of the head 42 of the stopper 10, 100, 200, 300. The annular fillet portion 58 may have a radius of curvature that is less than the radius r of curvature of the convex outer surface 44 of the head 42 of the stopper 10, 100, 200, 300.

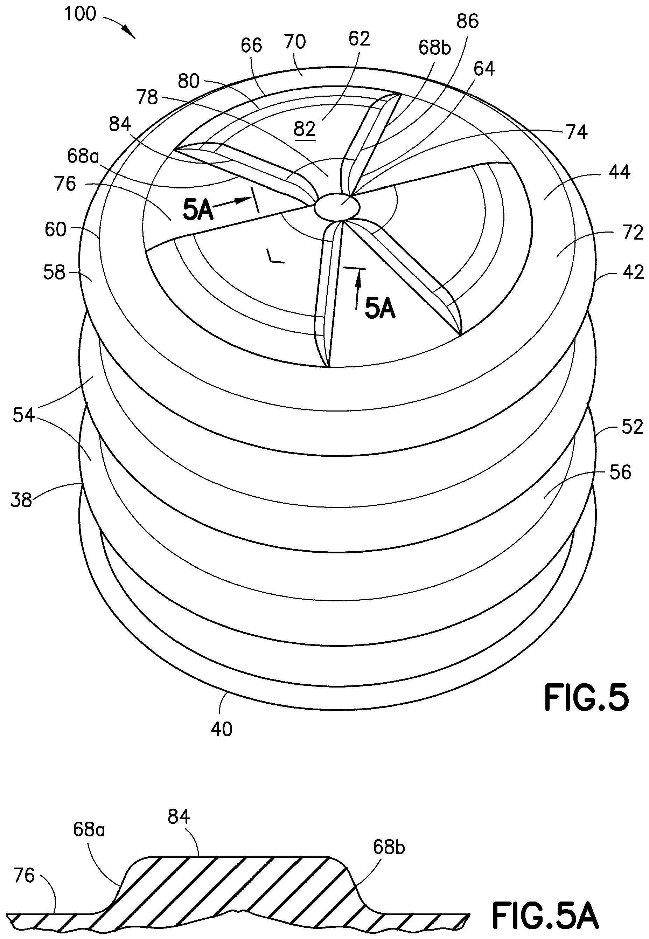

Optionally, as shown in FIGS. 5-10, anti-sticking features may be provided on the outer surface 44 of the head 42 of a stopper 100, 200, 300 having any combination of the above described features.

As shown in FIGS. 5-7, the optional anti-sticking features may be protrusions 62 extending outwardly from the convex outer surface 44 of the head 42 of the stopper 100 that decrease the contact area between the heads 42 of two stoppers 100 when the heads 42 of the two stoppers 100 come into contact with one another. The protrusions 62 may have a substantially triangular shape, preferably a shape that is substantially an isosceles triangle, with a vertex 64 corresponding to the vertex angle of the triangular shape of the protrusion 62, a base 66 corresponding to the base of the triangular shape of the protrusion 62, and two sides 68a, 68b extending between the vertex 64 and the base 66. The vertex 64 of the protrusion 62 has a circumferential width that is less than the circumferential width of the base 66 of the protrusion 62.

The vertex 64 of each protrusion 62 is adjacent the center of the outer surface 44 of the head 42 of the stopper 100, i.e., the center of origin of the outer perimeter 60 of the outer surface 44 of the head 42 of the stopper 100, which corresponds to the apex of the convex outer surface 44 of the head 42 of the stopper 100, and the base 66 of each protrusion 62 is adjacent the outer perimeter 60 of the outer surface 44 of the head 42 of the stopper 100.

The base 66 of the protrusions 62 may be rounded to correspond to the round outer perimeter 60 of the outer surface 44 of the head 42 of the stopper 100. Typically the radius of curvature of said base 66 of the protrusions 62 may be between 2.0 and 27.2% of the maximal diameter of the head of the stopper; preferably between 2.5 and 18.5%, or even more preferably between 5.5 and 13.0%.

A space 70 may be provided between the base 66 of the protrusions 62 and the outer perimeter 60 of the outer surface 44 of the head 42 of the stopper 100, such that the protrusions 62 are distanced from the outer perimeter 60 of the outer surface 44 of the head 42 of the stopper 100, and an annular portion 72 of the outer surface 44 of the head 42 of the stopper 100 directly adjacent to the outer perimeter 60 of the outer surface 44 of the head 42 of the stopper 100 is not covered by the protrusions 62.

The vertex 64 of the protrusions 62 may be truncated, such that a space 74 is provided at the center of the outer surface 44 of the head 42 of the stopper 100. The space 74 may be circular and the truncated vertex 64 of the protrusions 62 may be curved such that the vertex 64 of the protrusions 62 define the space 74 at the center of the outer surface 44 of the head 42 of the stopper 100 that is not covered by the protrusions 62.

The protrusions 62 are evenly distributed around the circumference of the outer surface 44 of the head 42 of the stopper 100 such that corresponding substantially triangular spaces 76 are provided between adjacent protrusions 62. The resulting spaces 76 between the protrusions 62, like the protrusions 62, are generally wedge or pie shaped. The angular distance θ_{S} occupied by the spaces 76, i.e. the angular distance between two adjacent protrusions 62, is less than the angular distance θ_{P} occupied by one of the protrusions 62, i.e., θ_{S} < θ_{P}.

Three to six (3-6) protrusions 62 may be provided on the outer surface 44 of the head 42 of the stopper 100. The angular distance θ_{P} occupied by one of the protrusions 62 may be 40-80°, preferably 50-80°, and more preferably, 55-75°. Examples are provided in Table 1.

The angular distance occupied by one protrusion 62 and one space 76 (θ_{P}+ θ_{S}) is 60-130°, preferably, 80-130°, and more preferably, 90-120°. Examples are provided in Table 1.

In FIGS. 5-7, θ_{P} is 72°, θ_{S} is 48°, and (θ_{P}+ θ_{S}) is 120°,

**Table 1**

| Number of Protrusions/Spaces | θ_{P} = 40° | | θ_{P} = 60° | | θ_{P} = 80° | |
|---|---|---|---|---|---|---|
| | θ_{S} | θ_{P}+ θ_{S} | θ_{S} | θ_{P}+ θ_{S} | θ_{S} | θ_{P}+ θ_{S} |
| 3 | ----- | ----- | ----- | ----- | 40 | 120 |
| 4 | ----- | ----- | 30° | 90° | 10° | 90° |
| 5 | 32° | 72° | 12° | 72° | ----- | ----- |
| 6 | 20° | 60° | ----- | ----- | ----- | ----- |

The radial length 1 of each protrusion 62 is 70-90%, preferably 75-80%, of the outer diameter d of the outer surface 44 of the head 42 of the stopper 100.

The outer surface of the protrusions 62 may be flat or concave or convex with a curvature extending from the vertex 64 to the base 66. The curvature of the protrusion 62 in this direction may have a single radius of curvature or multiple radiuses of curvature. When the curvature in this direction has multiple radiuses of curvature, a fillet portion 78 at the vertex 64 of the protrusion 62 may have a first radius of curvature, a fillet portion 80 at the base 66 of the protrusion 62 may have a second radius of curvature, and the upper surface 82 extending between the fillet portion 78 of the vertex 64 and the fillet portion 80 of the base 66 may be flat or have a third radius of curvature. The first radius of curvature and the second radius of curvature may be equal and/or the third radius of curvature may be greater than the first radius of curvature and the second radius of curvature. The curvature of the fillet portion 78 of the vertex 64 and/or the curvature of the fillet portion 80 of the base 66 may be concave, i.e., rounded inwardly, or convex, i.e. rounded outwardly. Preferably, the curvature of the fillet portion 78 of the vertex 64 and the fillet portion 80 of the base 66 have a convex curvature where the fillets abut the upper surface 82 that changes to a concave curvature as the fillet 78, 80 approaches the outer surface 44 of the head 42 of the stopper 100. Typically the radius of curvature of said fillet portion 78 may be comprised between 2.0 and 27.2% of the maximal diameter of the head of the stopper; preferably between 2.5 and 18.5%, or even more preferably between 5.5 and 13.0%.

Similarly, alternatively or in addition to any curvature of the outer surface of the protrusions 62 extending from the vertex 64 to the base 66, the outer surface of the protrusions 62 may be flat or concave or convex with a curvature extending from one side 68a of the protrusion 62 to the other side 68b of the protrusion 62. The curvature of the protrusion 62 in this direction may have a single radius of curvature or multiple radiuses of curvature. As shown in FIG. 5A, when the curvature in this direction has multiple radiuses of curvature, a fillet portion 84 at one side 68a of the protrusion 62 may have a first radius of curvature, a fillet portion 86 at the other side 68b of the protrusion 62 may have a second radius of curvature, and the upper surface 82 extending between the fillet portions 84, 86 at the sides 68a, 68b of the protrusion 62 may be flat or have a third radius of curvature. The first radius of curvature and the second radius of curvature may be equal and/or the third radius of curvature may be greater than the first radius of curvature and the second radius of curvature. The curvature of the fillet portions 84, 86, of the sides 68a, 68b of the protrusion 62 may be concave, i.e., rounded inwardly, and/or convex, i.e. rounded outwardly. Typically the radius of curvature of said fillet portion 84, 86 may be between 2.0 and 27.2% of the maximal diameter of the head of the stopper; preferably between 2.5 and 18.5%, or even more preferably between 5.5 and 13.0%.

Preferably, as shown in FIG. 5A, the fillet portions 84, 86 will have a convex curvature where the fillets 84, 86 abut the upper surface 82 that changes to a concave curvature as the fillet 84, 86 approaches the outer surface 44 of the head 42 of the stopper 100.

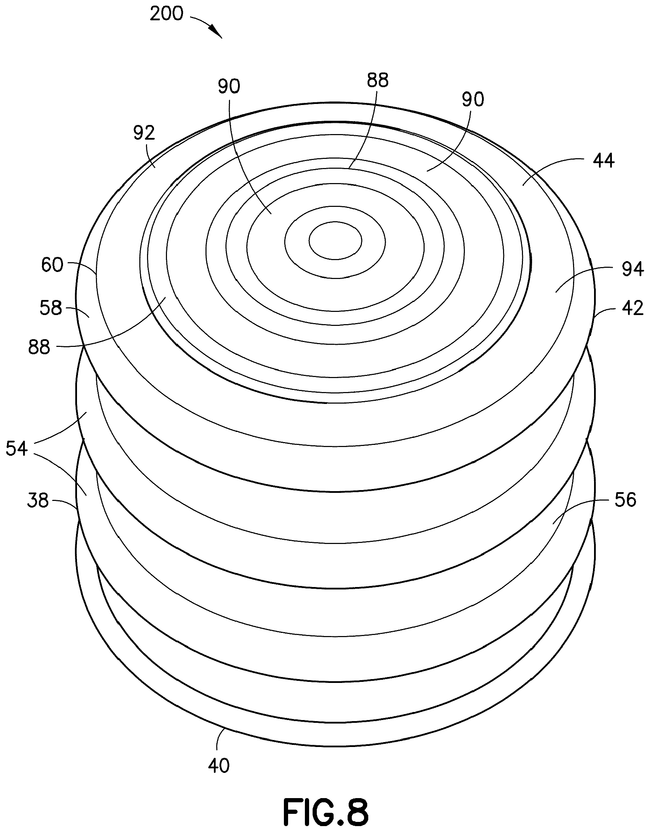

Alternatively, as shown in FIGS. 8-10, the optional anti-sticking features may be concentric annular protrusions 88 extending outwardly from the outer surface 44 of the head 42 of the stopper 200. The annular protrusions 88 are separated by interspaces 90. The outer surface of the annular protrusions 88 may have a convex curvature while the interspace 90 between adjacent annular protrusions 88 may have a concave curvature. The radius of curvature of the interspaces 90 may be greater than or equal to the radius of curvature of the outer surface of the annular protrusions 88 and is preferably greater than the radius of curvature of the outer surface of the annular protrusions 88. The radius of curvature of the annular protrusions 88 may be equal to or slightly greater than the radius of curvature of the annular ribs 54 provided on the outer sidewall 52 of the stopper 200.

Two to four (2-4) annular protrusions 88 may be provided on the outer surface 44 of the head 42 of the stopper 200, and preferably, three annular protrusions 88 are provided on the outer surface 44 of the head 42 of the stopper 200.

The radial width of the annular protrusions 88 may be the same or different and the radial width of the interspaces 90 between adjacent annular protrusions 88 may be the same or different.

A space 92 may be provided between the outermost annular protrusion 88 and the outer perimeter 60 of the outer surface 44 of the head 42 of the stopper 200, such that the outermost annular protrusion 88 is distanced from the outer perimeter 60 of the outer surface 44 of the head 42 of the stopper 200, and an annular portion 94 of the outer surface 44 of the head 42 of the stopper 200 directly adjacent to the outer perimeter 60 of the outer surface 44 of the head 42 of the stopper 200 has the radius of curvature of the outer surface 44 of the head 42 of the stopper 200.

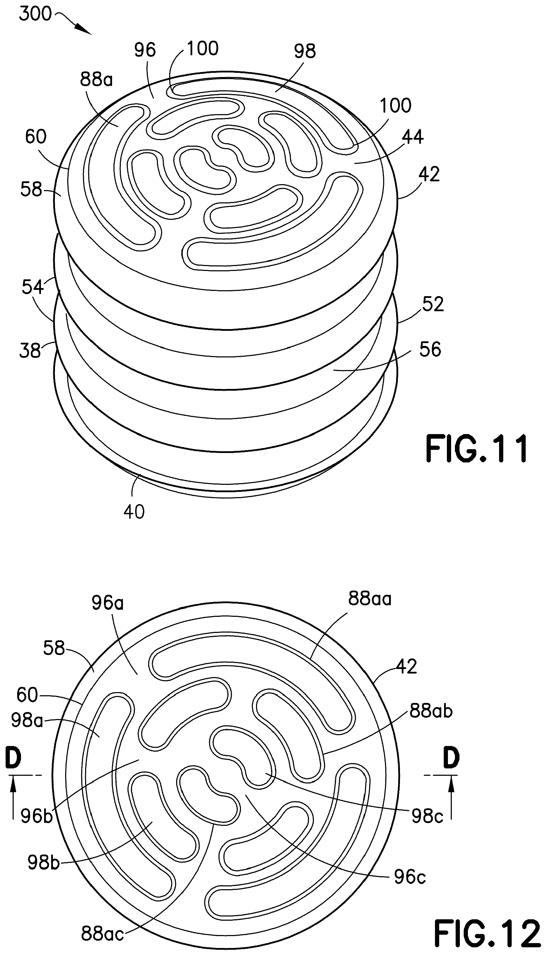

One or more of the annular protrusions 88 may be continuous as shown in FIGS. 8-10 and/or one or more of the annular protrusions 88a may be discontinuous as shown in FIGS. 11-13. The discontinuous annular protrusions 88a may have one gap 96, such that the annular protrusion is C-shaped or may have a plurality of gaps 96 (FIGS. 11-13) such that the annular protrusion 88a comprises a plurality of sections 98.

The number of gaps 96 provided in each of the concentric annular protrusions 88a may be the same or may differ. For example, as shown in FIGS. 11 and 12, the outermost annular protrusion 88aa may have three gaps 96a and three sections 98a, with the next inner annular protrusion 88ab having four gaps 96b and four sections 98b, and the innermost annular protrusion 88ac having two gaps 96c and two sections 98c. The gaps 96 in each of the concentric annular protrusions 88a may be positioned relative to one another such that none of the gaps 96 in one annular protrusion 88a correspond to the any gap 96 in any adjacent protrusion annular 88a and/or one or more of the gaps 96 in adjacent annular protrusions 88a may align with one another.

Each section 98 of the annular protrusions 88a may have a convex curvature in the radial direction and the ends 100 of each section 98 may be rounded. Typically the radius of curvature of said annular protrusions in the radial direction may be between 2.0 and 27.2% of the maximal diameter of the head of the stopper; preferably between 2.5 and 18.5%, or even more preferably between 5.5 and 13.0%.

The sections 98 of the annular protrusions 88a are sized and shaped such that, when the heads 42 of two stoppers 300 are in contact with one another, no section 98 of any of the annular protrusions 88a can be received in the gaps 96 of the annular protrusions 88a or the space between the annular protrusions 88a.

The sections 98 of the annular protrusions 88a may comprise 50-80% of the circumference of the annular protrusion 88a.

The inventive stopper solves the problem of head to head sticking of stoppers during storage prior to assembly into syringes and during assembly into syringes. Simultaneously, the convex outer surface of the head of the inventive stopper decreased the dead space remaining in the chamber and/or fitting of the syringe when an injection is completed and the stopper is compressed by the plunger rod into the bottom surface of the syringe body and reduces the contact area between the heads of two stoppers when the heads of the two stoppers come in contact with one another, thereby decreasing sticking of the stoppers to one another. When a coating and/or silicone layer are provided on the inventive stopper, the sticking is reduced without changing the composition and/or amount of the coating and/or silicone layer from that which is traditionally used on prior art stoppers.

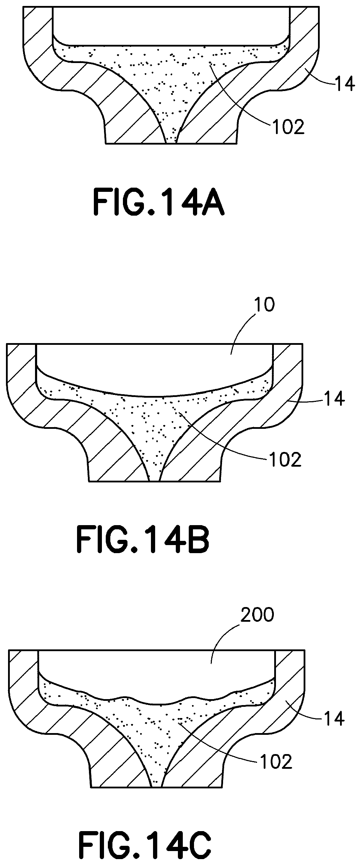

In addition, as shown in FIGS. 14A-14C, the inventive stopper (10, 100, 200, 300) reduces the amount of pharmaceutical composition 102 that remains in the syringe 12 after injection and cannot be ejected from the syringe body 14 no matter how high a force is exerted on the plunger rod 16 at the end of injection. This wasted pharmaceutical composition 102 is commonly referred to as the "dead volume" of the syringe 12.

Finite element analysis (FEA) conducted using Abaqus software (Table 1) shows that the inventive stopper 10, 200 having a convex head 42 (FIG. 14B) or having a convex head 42 with continuous annular protrusions 88 (FIG.14C) decreases the dead volume (mm³) as compared with a stopper having a flat head (FIG. 14A).

| | Force Applied to the Stopper (N) | | | | |
|---|---|---|---|---|---|
| Dead Volume (mm³) | 8.9 N | 15 N | 22.2 N | 25 N | 44.5 N |
| Flat | 18.2 mm³ | 16.5 mm³ | 14.9 mm³ | 14.4 mm³ | 11.5 mm³ |
| Convex | 11.6 mm³ | 10.1-11.6 mm³ | 9.0-11.6 mm³ | 8.6-11.6 mm³ | 7.2-11.6 mm³ |
| Convex with Annular Protrusions | 11.1 mm³ | 9.0-11.1 mm³ | 9.0-11.1 mm³ | 8.7-11.1 mm³ | 7.1-11.1 mm³ |

Whereas particular aspects of this invention have been described above for purposes of illustration, it will be evident to those skilled in the art that numerous variations of the details of the present invention may be made without departing from the invention.

## Claims

1. A stopper (10, 100, 200, 300) for a syringe (12) comprising:
a body (38) having a tail (40) adapted for attachment to a plunger rod (28) of the syringe (12) and a head (42),
**characterized in that**:
an outer surface (44) of the head (42) of the stopper (10, 100, 200, 300) is convex, such that the outer surface (44) of the head (42) of the stopper (10, 100, 200, 300) is curved with an apex of the curvature extending away from a plane orthogonal to the longitudinal axis (L) of the stopper (10, 100, 200, 300) and comprising an outer perimeter (60) of the outer surface (44), the apex of the outer surface (44) of the head (42) being higher than the outer perimeter (60) of the outer surface (44) when the stopper (10, 100, 200, 300) is viewed from a side in a direction perpendicular to the longitudinal axis (L) of the stopper (10, 100, 200, 300).

2. The stopper (10, 100, 200, 300) according to claim 1, wherein a ratio of a radius of curvature (r) of the outer surface (44) of the head (42) of the stopper (10, 100, 200) to a maximum diameter (D) of the head (42) of the stopper (10, 100, 200, 300) (r/D) is 1-20.

3. The stopper (10, 100, 200, 300) according to claim 1 or claim 2, further comprising a coating applied to at least a portion of the outer surface (44) of the head (40) of the stopper (10, 100, 200, 300) that will contact a pharmaceutical composition contained within the syringe (12).

4. The stopper (100) according to any of claims 1-3, further comprising a plurality of protrusions (62) extending outwardly from the outer surface (44) of the head (42) of the stopper (100), wherein the protrusions (62) preferably have a substantially triangular shape, more preferably a shape that is substantially an isosceles triangle, with a vertex (64) corresponding to the vertex angle of the triangular shape of the protrusion (62), a base (66) corresponding to the base of the triangular shape of the protrusion (62), and two sides (68a, 68b) extending between the vertex (64) and the base (66), wherein the vertex (64) of each protrusion (62) is adjacent the center of the outer surface (44) of the head (42) of the stopper (100) and the base (66) of each protrusion (62) is adjacent the outer perimeter (60) of the outer surface (44) of the head (42) of the stopper (100).

5. The stopper (100) according to claim 4, wherein a space (70) is provided between the bases (66) of the protrusions (62) and the outer perimeter (60) of the outer surface (44) of the head (42) of the stopper (100) and/or a space (74) defined by the vertex (64) of the protrusions (62) is provided at a center of the outer surface (44) of the head (42) of the stopper (100).

6. The stopper (100) according to claim 4 or claim 5, wherein the protrusions (62) are evenly distributed around a circumference of the outer surface (44) of the head (42) of the stopper (100) such that corresponding substantially triangular spaces (76) are provided between adjacent protrusions (62), and an angular distance θ_{S} occupied by the spaces (76) is less than the angular distance θ_{P} occupied by one of the protrusions (62), and, preferably, the angular distance θ_{P} occupied by one of the protrusions (62) is 40-80° and/or the angular distance occupied by one protrusion (62) and one space (76) (θ_{P}+ θ_{S}) is 60-130°.

7. The stopper (100) according to any of claims 4-6, wherein a radial length (1) of each protrusion (62) is 70-90% of an outer diameter (d) of the outer surface (44) of the head (42) of the stopper (100).

8. The stopper (100) according to any of claims 4-7, wherein an outer surface of the protrusions (62) is flat or concave or convex with a curvature extending from the vertex (64) to the base (66) and/or the outer surface of the protrusions (62) is flat or concave or convex with a curvature extending from one side (68a) of the protrusion (62) to the other side (68b) of the protrusion (62).

9. The stopper (100) according to claim 8, wherein the curvature extending from the vertex (64) to the base (66) of the protrusion (62) is defined by a fillet portion (78) at the vertex (64) of the protrusion (62) having a first radius of curvature, a fillet portion (80) at the base (66) of the protrusion (62) having a second radius of curvature, and an upper surface (82) extending between the fillet portion (78) at the vertex (64) and the fillet portion (80) at the base (66) that is flat or has a third radius of curvature, wherein the first radius of curvature and the second radius of curvature may be equal and/or the third radius of curvature may be greater than the first radius of curvature and the second radius of curvature; and/or the curvature extending from one side (68a) of the protrusion (62) to the other side (68b) of the protrusion (62) is defined by a fillet portion (84) at one side (68a) of the protrusion (62) having a first radius of curvature, a fillet portion (86) at the other side (68b) of the protrusion (62) having a second radius of curvature, and the upper surface (82) extending between the fillet portions (84, 86) at the sides (68a, 68b) of the protrusion (62) is flat or has a third radius of curvature, wherein the first radius of curvature and the second radius of curvature may be equal and/or the third radius of curvature may be greater than the first radius of curvature and the second radius of curvature.

10. The stopper (200, 300) according to any of claims 1-3, further comprising a plurality of concentric annular protrusions (88, 88a) extending outwardly from the outer surface (44) of the head (42) of the stopper (200, 300) and separated by interspaces (90).

11. The stopper (200, 300) according to claim 10, wherein an outer surface of the annular protrusions (88, 88a) has a convex curvature in the radial direction while the interspaces (90) between adjacent annular protrusions (88, 88a) have a concave curvature in the radial direction.

12. The stopper (200, 300) according to claim 10 or claim 11, wherein a radius of curvature of the interspaces (90) is greater than or equal to the radius of curvature of the outer surface of the annular protrusions (88, 88a) and is preferably greater than the radius of curvature of the outer surface of the annular protrusions (88, 88a).

13. The stopper (200, 300) according to any of claims 10-12, wherein a space (92) is provided between the outermost annular protrusion (88, 88a) and the outer perimeter (60) of the outer surface (44) of the head (42) of the stopper (200).

14. The stopper (300) according to any of claims 10-13, wherein the annular protrusions (88a) are discontinuous and include at least one gap (96) such that the annular protrusion (88a) comprises a plurality of sections (98).

15. A syringe (12) comprising:
a syringe body (14) defining a chamber (24);
a plunger rod (28) at least partially received within the chamber (24); and
a stopper (10, 100, 200, 300) according to any of claims 1-15 attached to an end of the plunger rod (28) received within the chamber (24).
